# FASCICULE DE BREVET EUROPEEN

(11) **EP 4 186 440 B1**
(45) Date de publication et mention de la délivrance du brevet: **31.07.2024**
(21) Numéro de dépôt: 22208999.7
(22) Date de dépôt: 23.11.2022
(51) Int. Cl.: A61B 17/00

(54) **SYSTÈME CHIRURGICAL ASSURANT LA CONNEXION RÉVERSIBLE ENTRE DEUX IMPLANTS OU ENTRE UN IMPLANT ET UN INSTRUMENT DU SYSTÈME CHIRURGICAL**
CHIRURGISCHES SYSTEM ZUR REVERSIBLEN VERBINDUNG ZWISCHEN ZWEI IMPLANTATEN ODER EINEM IMPLANTAT UND EINEM INSTRUMENT DES CHIRURGISCHEN SYSTEMS
SURGICAL SYSTEM PROVIDING A REVERSIBLE CONNECTION BETWEEN TWO IMPLANTS OR BETWEEN AN IMPLANT AND A TOOL OF THE SURGICAL SYSTEM

(30) Priorité: 24.11.2021 FR 2112449
(43) Date de publication de la demande: 31.05.2023
(73) Titulaire: Imprint Medical, 69530 Brignais (FR); Euclide Care, 87700 Aixe sur Vienne (FR)
(72) Inventeur: BREUILH, Christophe David, 87350 PANAZOL (FR); GUILLON-COTTARD, Didier, Julien, 69600 OULLINS (FR)
(74) Mandataire: Lavoix

(56) Documents cités:
- WO-A1-2020/206316
- WO-A2-2008/039981
- US-A1- 2007 233 249
- US-A1- 2009 319 043

## Description

La présente invention concerne un système chirurgical comprenant deux éléments, qui sont soit deux implants chirurgicaux, soit un implant chirurgical et un instrument chirurgical, et qui intègrent des aménagements respectifs assurant leur connexion réversible.

Lors de diverses opérations mises en oeuvre en lien avec une intervention chirurgicale, il est nécessaire de connecter l'un à l'autre, de façon réversible, soit des implants chirurgicaux, soit un implant chirurgical et un instrument chirurgical. Par exemple, lors de la pose d'un implant chirurgical dans le corps d'un patient, le chirurgien utilise un ancillaire de manière à, successivement, connecter l'implant à l'ancillaire, positionner, le cas échéant en force, l'implant dans le corps du patient en manipulant l'ancillaire, puis désolidariser de l'ancillaire l'implant en place. En pratique, on connaît diverses formes de réalisation pour les aménagements respectifs des deux éléments chirurgicaux, assurant la connexion réversible entre ces derniers.

Une de ces formes de réalisation, couramment répandue, consiste en une liaison filetée entre les éléments chirurgicaux : l'un des deux éléments intègre ainsi un mécanisme ayant une tige saillante filetée, tandis que l'autre élément est pourvu d'un trou taraudé dans lequel la tige filetée est à visser pour assurer la connexion entre les deux éléments. Une telle liaison filetée n'est toutefois pas satisfaisante à plusieurs égards. D'abord, le chirurgien est contraint, pendant le vissage de la tige filetée dans le trou taraudé, de maintenir en place les deux éléments, tout en entraînant la tige en rotation sur plusieurs tours. Les gestes correspondants du chirurgien sont donc relativement compliqués, en mobilisant généralement ses deux mains, et prennent un temps important en peropératoire. Ensuite, la liaison filetée court le risque de ne pas être suffisamment sécurisée : lors de l'intervention chirurgicale, les deux éléments risquent de se désolidariser de manière intempestive et/ou la connexion entre eux risque de se casser en cas d'application d'efforts importants, par exemple en flexion ou en impaction. Enfin, une telle liaison filetée est contraignante à réaliser par fabrication additive : lorsque les éléments chirurgicaux sont obtenus par fabrication additive, cette dernière ne permet généralement pas, par manque de précision, d'obtenir directement le filetage et le taraudage de la liaison filetée, si bien qu'il est nécessaire de retoucher les éléments fabriqués, en mettant en oeuvre des opérations de reprise ou de retouche conventionnelles, par exemple des opérations d'usinage à l'outil. Ces opérations de retouche ou de reprise supplémentaires peuvent être complexes à mettre en oeuvre, risquent de contaminer les éléments chirurgicaux, par exemple par des huiles de coupe, et induisent un coût supplémentaire significatif.

WO 2020/206316 divulgue un système chirurgical dans lequel un implant, à savoir une attache chirurgicale, et un instrument sont connectables de manière réversible par une liaison hélicoïdale formée par deux bras en spirale de l'implant et deux tiges en spirale de l'instrument. La connexion entre l'implant et l'instrument est commandée par une gâchette qui fait s'engrener les bras en spirale et les tiges en spirale, sans pour autant permettre de verrouiller la liaison hélicoïdale formée par ces derniers.

Le but de la présente invention est de proposer un nouveau système chirurgical dont la connexion réversible de deux éléments soit à la fois plus simple à opérer, plus sécurisée, et moins contraignante à fabriquer, en particulier quand les éléments sont réalisés par fabrication additive.

A cet effet, l'invention a pour objet un système chirurgical, tel que défini à la revendication 1.

Une des idées à la base de l'invention est de chercher à assurer la connexion des deux éléments du système chirurgical non pas par une liaison filetée, mais par une liaison hélicoïdale dont les surfaces hélicoïdales qui la forment n'ont pas à être un filetage et un taraudage conventionnels. Dans le système conforme à l'invention, la liaison hélicoïdale est formée par deux surfaces hélicoïdales qui sont respectivement délimitées par l'un et l'autre des deux éléments, en s'étendant depuis des faces d'appui respectives des deux éléments. De plus, le système conforme à l'invention comprend un mécanisme, qui est intégré à l'un ou l'autre des deux éléments et qui est conçu pour être actionné selon deux mouvements simples et rapides à mettre en oeuvre par le chirurgien, typiquement d'une seule main, à savoir un premier mouvement qui est translatif suivant l'axe de la liaison hélicoïdale, sans être rotatif autour de cet axe, et un second mouvement qui est rotatif autour de cet axe, en étant potentiellement combiné à une composante translative selon cet axe. Le mécanisme et les deux surfaces hélicoïdales de la liaison hélicoïdale sont configurés de sorte que, lorsque le mécanisme est actionné selon le premier mouvement, le mécanisme agit sur la liaison hélicoïdale en faisant s'engrener les deux surfaces hélicoïdales l'une avec l'autre jusqu'à mettre en butée axiale les faces d'appui précitées des deux éléments, puis lorsque le mécanisme est actionné selon le second mouvement, le mécanisme agit sur la liaison hélicoïdale pour la verrouiller tandis que les deux faces d'appui sont appuyées fermement l'une contre l'autre suivant l'axe, ce qui sécurise la connexion entre les deux éléments. Dans la mesure où les deux surfaces hélicoïdales n'ont pas à être réalisées sous forme d'un filet conventionnel et d'un taraudage conventionnel, qui formeraient une liaison filetée, ces deux surfaces hélicoïdales et, plus généralement, les deux éléments du système peuvent être réalisés par fabrication additive sans nécessiter de retouche ou de reprise, notamment par usinage. En pratique, comme les deux éléments chirurgicaux du système selon l'invention sont soit deux implants, soit un implant et un instrument, la liaison hélicoïdale et le mécanisme agissant sur cette dernière confèrent à ces éléments une grande adaptabilité d'usage, en lien notamment avec le patient opéré et/ou l'abord chirurgical et/ou la technique chirurgicale.

Des caractéristiques additionnelles avantageuses du système chirurgical conforme à l'invention sont spécifiées aux autres revendications.

L'invention sera mieux comprise à la lecture de la description qui va suivre, donnée uniquement à titre d'exemple et faite en se référant aux dessins sur lesquels :
- la figure 1 est une vue en perspective d'un mode de réalisation d'un système chirurgical conforme à l'invention ;
- la figure 2 est une vue similaire à la figure 1, illustrant partiellement le système chirurgical sous un angle de vue différent de celui de la figure 1 ;
- la figure 3 regroupe un insert a), qui est une coupe longitudinale du système de la figure 1, un insert b), qui est une coupe selon la ligne b-b indiquée sur l'insert a), et un insert c), qui est une coupe selon la ligne c-c indiquée sur l'insert a) ;
- les figures 4 et 5 sont des vues similaires à la figure 3, les figures 3 à 5 illustrant respectivement trois configurations successivement mises en oeuvre lors de l'utilisation du système chirurgical ;
- la figure 6 est une vue en perspective illustrant diverses variantes de réalisation pour une partie du système chirurgical ;
- la figure 7 est une vue en élévation illustrant également diverses formes de réalisation pour une partie du système chirurgical ; et
- les figures 8 et 9 sont des vues en perspective, sous des angles d'observation différents, d'un second mode de réalisation du système chirurgical conforme à l'invention.

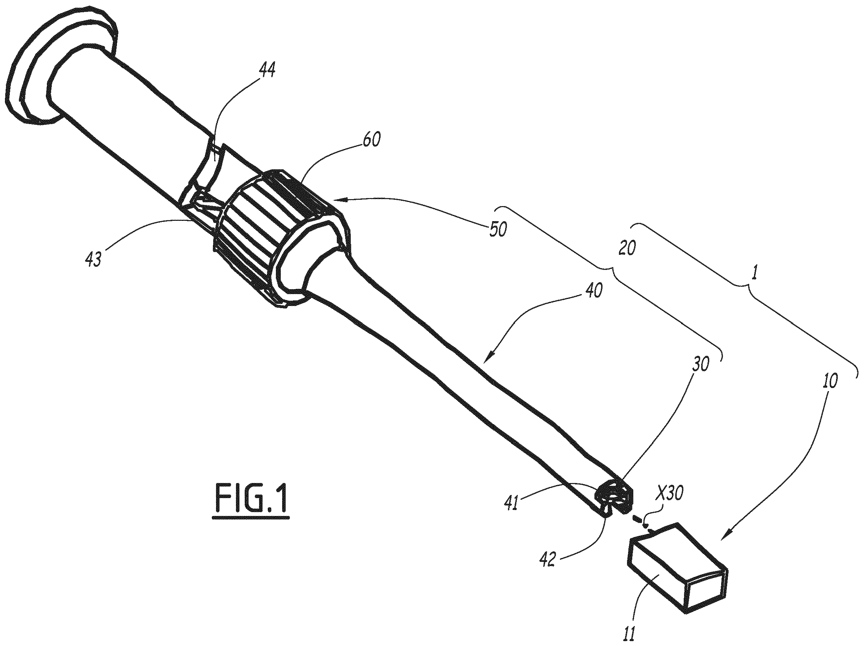

Sur les figures 1 à 5 est représenté un système chirurgical 1 comprenant un implant 10 et un instrument 20. Comme détaillé par la suite, l'implant 10 et l'instrument 20 intègrent des aménagements permettant de les connecter directement l'un à l'autre, et ce de manière réversible.

L'implant 10 comporte un corps 11 qui, aux fins de sa connexion à l'instrument 20, délimite une face d'appui 12, ainsi qu'une surface hélicoïdale 13 s'étendant en creux depuis la face d'appui 12. Sur les figures, le corps 11 est, en dehors de sa face d'appui 12 et de sa surface hélicoïdale 13, représenté de manière très schématique, sans illustrer la multitude de formes de réalisation envisageables en lien avec la finalité chirurgicale de l'implant 10. En pratique, les spécificités de l'implant 10, qui sont en lien avec ses fonctions autres que celle de sa connexion réversible à l'instrument 20, ne sont pas limitatives, si bien que le corps 11 de l'implant peut présenter, en dehors de sa face d'appui 12 et de sa surface hélicoïdale 13, de multiples et divers aménagements, non illustrés sur les figures.

Dans tous les cas, la surface hélicoïdale 13 est centrée sur un axe géométrique X13 autour duquel cette surface hélicoïdale s'enroule, en s'étendant à l'intérieur du corps 11 depuis la face d'appui 12. La surface hélicoïdale 13 forme ainsi, à l'intérieur du corps 11, une cavité 14, qui est centrée sur l'axe X13 et qui débouche axialement sur la face d'appui 12. Cette cavité 14 fait que l'implant 10 peut être qualifié d'élément femelle.

Suivant une disposition avantageuse dont l'intérêt apparaîtra plus loin, le corps 11 de l'implant 10 comporte également un ou plusieurs reliefs en creux 15, ici en deux exemplaires, distincts de la cavité 14. Dans l'exemple de réalisation considéré sur les figures, ces reliefs en creux 15 s'étendent depuis la face d'appui 12 du corps 11. Chaque relief en creux 15 occupe une portion du corps 11 autour de l'axe X13, les deux reliefs en creux 15 étant ici diamétralement opposés l'un à l'autre par rapport à l'axe X13.

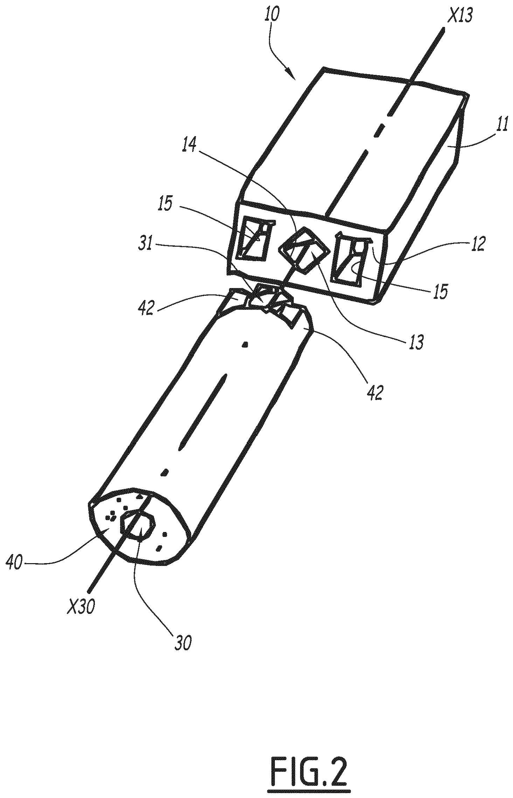

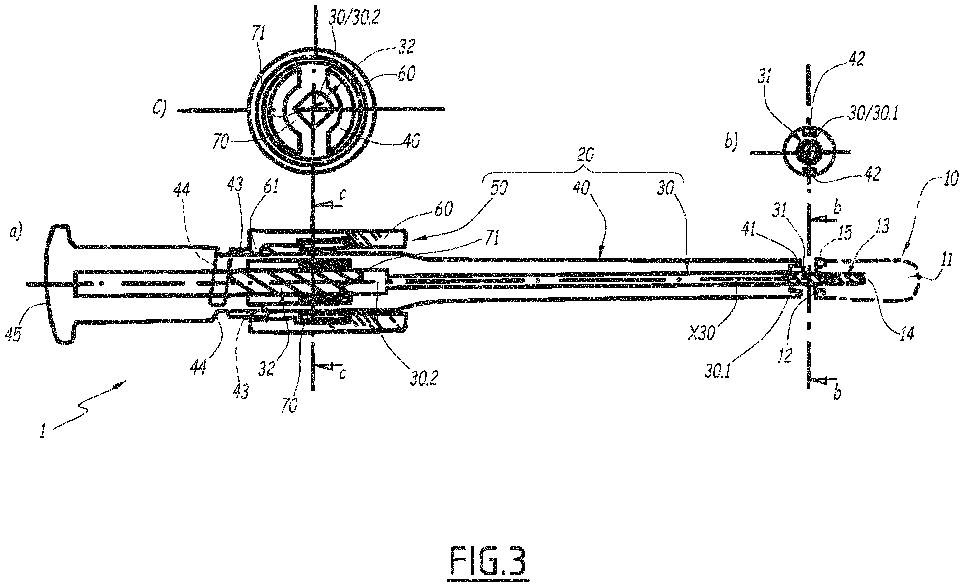

L'instrument 20 comporte, quant à lui, un arbre 30, qui est bien visible sur les figures 3 à 5 et qui n'est que marginalement visible sur les figures 1 et 2.

L'arbre 30 définit un axe géométrique X30, sur lequel l'arbre est centré et le long duquel l'arbre s'étend en longueur. Comme bien visible sur les figures 3 à 5, l'arbre 30 délimite extérieurement, c'est-à-dire sur sa face latérale extérieure, deux surfaces hélicoïdales 31 et 32 distinctes l'une de l'autre, qui sont centrées sur l'axe X30 et qui s'enroulent autour de l'arbre 30, en y formant des parties respectives de sa face latérale extérieure. Les surfaces hélicoïdales 31 et 32 occupent des parties longitudinales respectives 30.1 et 30.2, qui sont distinctes l'une de l'autre, en étant réparties sur l'arbre 20 suivant l'axe X30. La partie longitudinale 30.1, qui délimite la surface hélicoïdale 31, constitue une partie longitudinale terminale de l'arbre 20, tandis que la partie longitudinale 30.2, qui délimite la surface hélicoïdale 32, constitue soit une partie longitudinale terminale de l'arbre, axialement opposée à la partie longitudinale 30.1, soit une partie longitudinale intermédiaire de l'arbre.

La surface hélicoïdale 31 est congruente à la surface hélicoïdale 13 de l'implant 10 de sorte que, lorsque les surfaces hélicoïdales 31 et 13 sont rendues coaxiales l'une avec l'autre et entraînées en rotation relative autour de leur axe X30 et X13, ces surfaces hélicoïdales 31 et 13 s'engrènent l'une avec l'autre, induisant leur déplacement axial relatif, et ce de manière réversible. Autrement dit, les surfaces hélicoïdales 31 et 13 forment une liaison hélicoïdale entre l'implant 10 et l'instrument 20, plus précisément entre le corps 11 de l'implant et l'arbre 30 de l'instrument.

L'instrument 20 comporte également un support 40 sur lequel l'arbre 21 est monté de façon mobile, comme détaillé ci-après.

Dans l'exemple de réalisation considéré sur les figures, le support 40 présente une forme globalement tubulaire, à l'intérieur de laquelle est monté coaxialement l'arbre 30. Quelle que soit la forme du support 40, l'arbre 30 est monté sur ce dernier à la fois de manière fixe suivant l'axe X30 et de manière librement rotative autour de l'axe X30. Autrement dit, l'arbre 30 et le support 40 sont liés fixement l'un à l'autre en translation suivant l'axe X30, tout en étant libres en rotation autour de cet axe l'un par rapport à l'autre. En pratique, diverses formes de réalisation sont envisageables pour assurer cette liaison entre l'arbre 30 et le support 40. Dans l'exemple, non limitatif, envisagé sur les figures, l'arbre 30 présente des surfaces épaulées qui sont disposées en butée axiale contre des surfaces épaulées complémentaires, ménagées à l'intérieur de la paroi tubulaire du support 40, sans que la face intérieure de cette paroi tubulaire n'interfère avec l'arbre 30 suivant une direction périphérique à l'axe X30. En pratique, les mouvements rotatifs relatifs entre l'arbre 30 et le support 40 sont avantageusement guidés, par exemple par un ou plusieurs paliers ou des aménagements similaires, de manière à fixer la position de l'axe X30 par rapport au support 40 lorsque l'arbre 30 est monté sur le support.

De plus, quelle que soit la forme de réalisation du support 40, ce dernier délimite une face d'appui 41. A l'état monté de l'arbre 30 sur le support 40, cette face d'appui 41 est agencée transversalement à l'axe X30 et la partie longitudinale terminale 30.1 de l'arbre 30 émerge axialement de cette face d'appui 41, comme bien visible sur les figures 1 à 3. Ainsi, la surface hélicoïdale 31 s'étend depuis la face d'appui 41, en étant agencée en saillie de cette dernière, ce qui fait que l'instrument 20 peut être qualifié d'élément mâle.

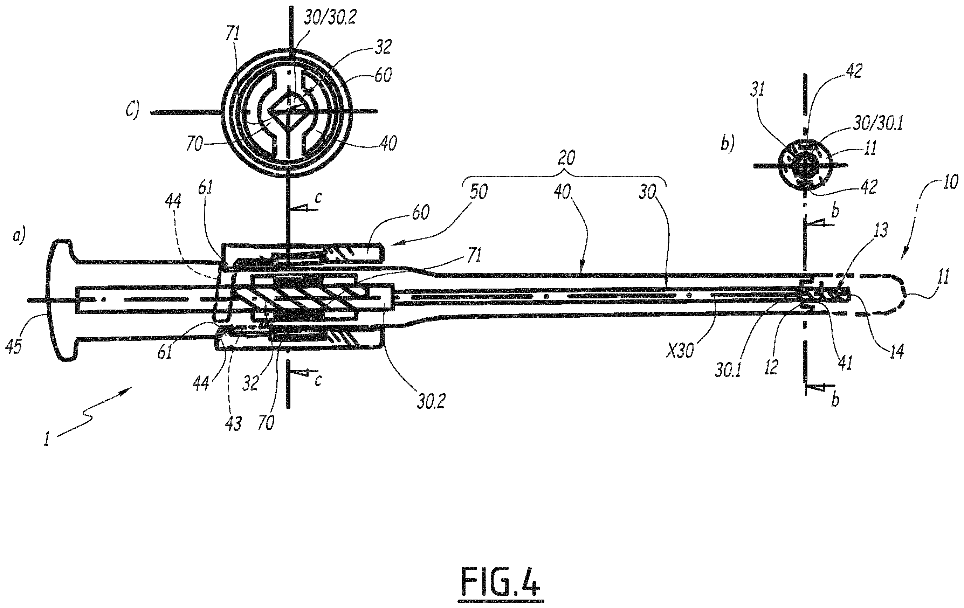

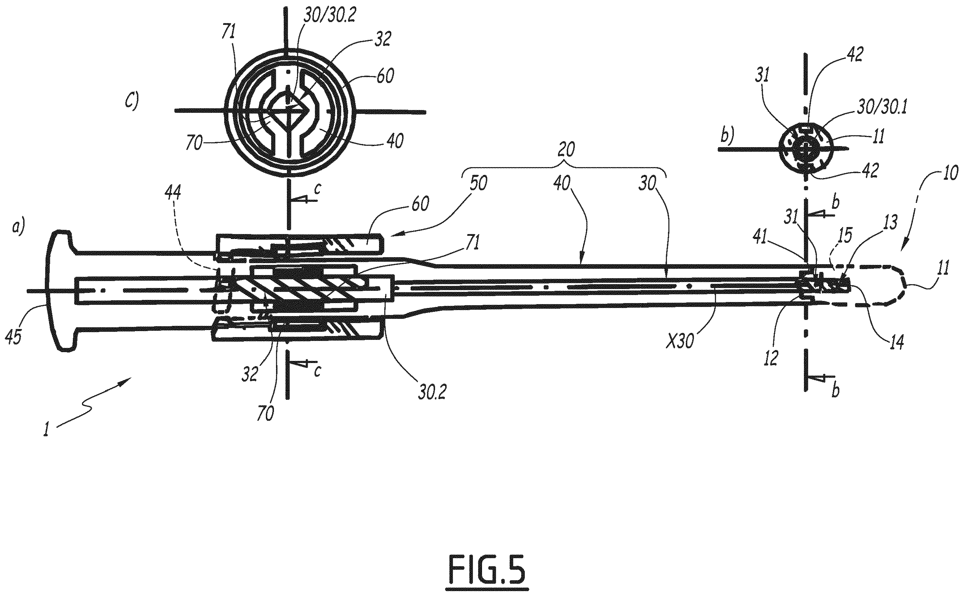

La face d'appui 41 est complémentaire de la face d'appui 12 de l'implant 10. Dès lors, lorsque les surfaces hélicoïdales 31 et 13 s'engrènent l'une avec l'autre de manière que leur déplacement axial, induit par leur engrènement, rapproche axialement l'un de l'autre l'implant 10 et l'instrument 20, les faces d'appui 41 et 12 se rapprochent axialement l'une de l'autre jusqu'à coopérer l'une avec l'autre par complémentarité de formes : le rapprochement axial précité entre les faces d'appui 41 et 12 aboutit à ce que ces faces d'appui 41 et 12 se retrouvent juxtaposées l'une contre l'autre comme sur la figure 4, puis sont pressées axialement l'une contre l'autre, comme sur la figure 5, en étant alors appuyées fermement l'une contre l'autre, ce qui induit notamment un effet de solidarisation rigide entre le support 40 et l'implant 10, par contact frottant entre les faces d'appui 41 et 12, et ce qui tend à supprimer tout jeu résiduel entre ces faces d'appui.

Suivant une disposition avantageuse, le support 40 comporte également un ou plusieurs reliefs en saillie 42, ici au nombre de deux. Ces reliefs en saillie 42 sont complémentaires des reliefs en creux 15 de l'implant 10 de sorte que, lorsque les surfaces hélicoïdales 31 et 13 s'engrènent l'une avec l'autre de manière à rapprocher l'une de l'autre les faces d'appui 41 et 12, les reliefs en saillie 42 coopèrent par complémentarité de formes avec les reliefs en creux 15 de sorte à bloquer en rotation autour de l'axe X30 le support 40 et l'implant 10 l'un par rapport à l'autre. Cette coopération par complémentarité de formes, entre les reliefs en saillie 42 et les reliefs en creux 15 consiste, ici, à ce que les reliefs en saillie 42 soient axialement reçus à l'intérieur des reliefs en creux 15 de manière ajustée entre eux, comme illustré schématiquement sur les figures 4 et 5. On comprend que les spécificités géométriques des reliefs en saillie 42 et des reliefs en creux 15 ne sont pas limitatives du moment que ces reliefs en saillie 42 et ces reliefs en creux 15 sont façonnés pour coopérer par complémentarité de formes aux fins du blocage en rotation autour de l'axe X30 du support 40 et de l'implant 10 l'un par rapport à l'autre. Suivant une forme de réalisation pratique, qui est mise en oeuvre dans l'exemple illustré aux figures, les reliefs en saillie 42 s'étendent depuis la face d'appui 41.

L'instrument 20 comporte en outre un mécanisme 50 permettant de commander la connexion réversible entre l'implant 10 et l'instrument 20. Le mécanisme 50 est conçu pour agir sur la liaison hélicoïdale entre l'implant 10 et l'instrument 20, formée par les surfaces hélicoïdales 13 et 31, comme expliqué ci-après.

Dans le mode de réalisation envisagé sur les figures, le mécanisme 50 comporte une bague d'actionnement 60 et un tiroir d'entraînement 70.

La bague d'actionnement 60 est montée sur le support 40 en étant guidée en déplacement par rapport au support 40 suivant deux mouvements distincts qui sont mis en oeuvre de manière successive lors de l'actionnement du mécanisme 50, à savoir :
- un premier mouvement, par lequel la bague d'actionnement 60 passe, par rapport au support 40, de sa position de la figure 3 à sa position de la figure 4, et qui est translatif suivant l'axe X30 sans être rotatif autour de cet axe, et
- un second mouvement, par lequel la bague d'actionnement 60 passe, par rapport au support 40, de sa position de la figure 4 à sa position de la figure 5, et qui est, de manière combinée, rotatif autour de l'axe X30 et translatif suivant cet axe.

Dans l'exemple envisagé aux figures, la bague d'actionnement 60 ceinture la paroi tubulaire du support 40, au niveau d'une partie intermédiaire de ce dernier suivant l'axe X30. Aux fins du guidage en déplacement de la bague d'actionnement 60 par rapport au support 40 suivant les premier et second mouvements, la face intérieure de la bague d'actionnement 60 coopère par contact avec la face extérieure de la paroi tubulaire du support 40 : à cet effet, la face intérieure de la bague d'actionnement 60 est, par exemple, pourvue d'un ou plusieurs pions saillants 61, ici en deux exemplaires diamétralement opposés suivant la périphérie intérieure de la bague d'actionnement 60, ces pions saillants 61 étant respectivement engagés dans des chemins de guidage, délimités par la face extérieure de la paroi tubulaire du support 40 et incluant chacun à la fois une glissière rectiligne 43, qui s'étend parallèlement à l'axe X30 et qui guide la bague d'actionnement 60 suivant le premier mouvement, et une came 44, qui s'enroule autour de l'axe X30 et qui guide la bague d'actionnement 60 suivant le second mouvement. Pour des raisons qui apparaitront plus loin, les cames 44 présentent de préférence un angle d'hélice supérieur à 84° environ de sorte que la composante translative du second mouvement présente une valeur bien moindre que celle de la composante rotative du second mouvement ; cela revient à dire que, lorsque la bague d'actionnement 60 parcourt sa course totale suivant le second mouvement, elle effectue majoritairement un mouvement rotatif autour de l'axe X30 et marginalement un mouvement translatif selon cet axe. Par ailleurs, suivant un dimensionnement préférentiel, chaque came 44 s'enroule autour de l'axe X30 sur moins de 360°, voire de préférence moins de 180°, si bien que lorsque la bague d'actionnement 60 parcourt sa course totale suivant le second mouvement, elle effectue moins d'un tour sur elle-même, voire de préférence moins d'un demi-tour sur elle-même : de cette façon, lors de l'actionnement du mécanisme 50, l'utilisateur peut entraîner, par une seule main et d'un seul geste, la bague d'actionnement 60 afin que cette dernière parcourt toute sa course suivant le premier mouvement puis le second mouvement.

La forme de réalisation qui vient d'être décrite pour les chemins de guidage associant les glissières rectilignes 43 et les cames 44 n'est pas limitative. De multiples formes de réalisation sont envisageables pour assurer le guidage en déplacement de la bague d'actionnement 60 par rapport au support 40 suivant successivement les premier et second mouvements, voire, de manière plus générale, pour assurer l'actionnement du mécanisme 50 suivant successivement les premier et second mouvements, notamment avec le second mouvement qui est majoritairement rotatif et dont la course angulaire totale est inférieure à 360°, de préférence inférieure à 180°.

Le tiroir d'entraînement 70 est, quant à lui, monté sur le support 40 à la fois de manière fixe en rotation autour de l'axe X30 et de manière librement translative suivant cet axe. Le tiroir d'entraînement 70 est ainsi lié en rotation autour de l'axe X30 au support 40 tout en étant libre en translation suivant l'axe X30 par rapport au support 40. A cet effet, dans l'exemple de réalisation considéré sur les figures, le tiroir d'entraînement 70 est axialement enfilé sur le support 40, en étant disposé radialement de part et d'autre de la paroi tubulaire du support 40, étant noté que, comme bien visible sur les inserts c) des figures 3 à 5, le tiroir d'entraînement 70 inclut des portions transversales qui sont reçues dans des passages traversants de la paroi annulaire du support 40, ce qui bloque le tiroir d'entraînement 70 en rotation autour de l'axe X30 par rapport au support 40. Bien entendu, de multiples autres formes de réalisation sont envisageables à cet égard.

De plus, le tiroir d'entraînement 70 est monté sur la bague d'actionnement 60 de manière à être lié en translation suivant l'axe X30 à cette bague d'actionnement 60 tout en étant libre en rotation autour de l'axe X30 par rapport à la bague d'actionnement. A cet effet, dans l'exemple envisagé sur les figures, le tiroir d'entraînement 70 est monté à l'intérieur de la bague d'actionnement 60, en étant reçu dans une encoche délimitée par la périphérie intérieure de la bague d'actionnement de manière à y être librement rotatif par rapport à la bague d'actionnement tout en étant en butée axiale contre les extrémités axiales de l'encoche précitée. Là encore, bien entendu, de multiples formes de réalisation, alternatives à celle qui vient d'être décrite, sont envisageables à cet égard.

En outre, le tiroir d'entraînement 70 coopère par contact avec l'arbre 30 de manière à entraîner cet arbre 30 en rotation autour de l'axe X30 par rapport au support 40 lorsque le tiroir d'entraînement 70 est déplacé en translation suivant l'axe X30 par rapport au support 40, autrement dit lorsque la bague d'actionnement 60 est entraînée en translation suivant l'axe. A cet effet, dans l'exemple envisagé sur les figures, le tiroir d'entraînement 70 délimite, ici intérieurement, une surface hélicoïdale 71 qui est congruente à la surface hélicoïdale 32 de l'arbre 30 de sorte que, lorsque les surfaces hélicoïdales 71 et 32 sont rendues coaxiales l'une avec l'autre et entraînées en rotation relative autour de l'axe X30, ces surfaces hélicoïdales 71 et 32 s'engrènent l'une avec l'autre, induisant leur déplacement axial relatif, et ce de manière réversible. Autrement dit, les surfaces hélicoïdales 32 et 71 forment une liaison hélicoïdale entre l'arbre 30 et le tiroir d'entraînement 70, autrement dit, de manière plus générale, une liaison hélicoïdale entre l'arbre 30 et le mécanisme 50.

Là encore, bien que soient préférées des formes de réalisation, telles que celle décrite ci-dessus, dans lesquelles est prévue une liaison hélicoïdale entre l'arbre 30 et le mécanisme 50, d'autres formes de réalisation sont envisageables pour assurer l'entraînement en rotation de l'arbre 30 autour de l'axe X30 par rapport au support 40 lorsque le mécanisme 50 est actionné en translation suivant l'axe X30.

D'autres caractéristiques et avantages du système chirurgical 1 apparaîtront dans la description ci-dessous d'un exemple d'utilisation de ce système chirurgical pour connecter directement l'un à l'autre l'implant 10 et l'instrument 20. Cette utilisation est typiquement opérée par un chirurgien en peropératoire.

On considère initialement que l'implant 10 et l'instrument 20 sont séparés l'un de l'autre. Un chirurgien manipule alors l'instrument 20 de manière à aligner les axes X30 et X13 et à introduire axialement l'extrémité libre de la partie longitudinale terminale 30.1 de l'arbre 30 à l'intérieur de la cavité 14 de l'implant 10. Le système chirurgical 1 est alors dans la configuration illustrée à la figure 3. Dans cette configuration de la figure 3, la bague d'actionnement 60 occupe une position correspondant au point de départ de sa course totale suivant les premier et second mouvements successifs décrits plus haut. Autrement dit, ici, les pions 61 de la bague d'actionnement 60 sont respectivement situés à l'extrémité axiale des glissières rectilignes 43, opposée aux cames 44.

Le chirurgien actionne alors le mécanisme 50 suivant le premier mouvement précité, faisant passer le système chirurgical 1 de la configuration de la figure 3 à la configuration de la figure 4. Plus précisément, le chirurgien déplace la bague d'actionnement 60 suivant le premier mouvement, c'est-à-dire exclusivement en translation suivant l'axe X30, ce qui entraîne en translation le tiroir d'entraînement 70 de manière correspondante et, par-là, entraîne l'arbre 30 en rotation autour de l'axe X30. Cette mise en rotation de l'arbre 30 induit l'engrènement de sa surface hélicoïdale 31 avec la surface hélicoïdale 13 de l'implant 10 et, par-là, le déplacement axial de l'implant 10 vers l'instrument 20, en rapprochant axialement l'une de l'autre les faces d'appui 12 et 41. Le déplacement axial relatif entre l'implant 10 et l'instrument 20 induit ici également la mise en coopération des reliefs en creux 15 et des reliefs en saillie 42, ce qui, sans intervention complémentaire du chirurgien, bloque en rotation autour de l'axe X30 l'implant 10 et l'instrument 20 l'un par rapport à l'autre, afin d'éviter un engrènement inefficace des surfaces hélicoïdales 31 et 13.

Lorsque la bague d'actionnement 60 a parcouru toute sa course rectiligne selon le premier mouvement, c'est-à-dire, ici, lorsque les pions 61 de cette bague d'actionnement atteignent l'extrémité des glissières rectilignes 43, tournée vers les cames 44, le système chirurgical 1 est dans la configuration de la figure 4. Dans cette configuration, les faces d'appui 12 et 41 sont juxtaposées axialement l'une contre l'autre, en formant une interface de contact qui est agencée transversalement à l'axe X30. Ainsi, lorsque le mécanisme 50 est actionné suivant le premier mouvement, le mécanisme 50 fait s'engrener les surfaces hélicoïdales 13 et 31 l'une avec l'autre jusqu'à juxtaposer axialement les faces d'appui 12 et 41 l'une contre l'autre.

Le chirurgien actionne alors le mécanisme 50 suivant le second mouvement précité, faisant passer le système chirurgical de la configuration de la figure 4 à la configuration de la figure 5. Plus précisément, le chirurgien déplace la bague d'actionnement 60 selon le second mouvement, c'est-à-dire, de manière combinée, majoritairement en rotation autour de l'axe X30 et marginalement en translation selon l'axe X30. La composante rotative du second mouvement décale angulairement la bague d'actionnement 60 par rapport au support 40, ce qui empêche le déplacement axial de la bague d'actionnement dans un sens opposé à celui du premier mouvement et, par-là, ce qui empêche que les surfaces hélicoïdales 13 et 31 se désengrènent. Dans le même temps, la composante translative du second mouvement induit, suivant les mêmes considérations que celles développées ci-dessus pour le premier mouvement, un engrènement additionnel des surfaces hélicoïdales 13 et 31 et, par-là, une mise en pression axiale des faces d'appui 12 et 41 l'une contre l'autre ; autrement dit, l'arbre 30 se retrouve mis en tension suivant l'axe X30, en tractant axialement l'implant 10 en appui serré contre le support 40 de l'instrument 20 au niveau de l'interface de contact entre les faces d'appui 12 et 41.

Lorsque la bague d'actionnement 60 a parcouru la course selon le second mouvement, c'est-à-dire, ici, lorsque les pions 61 de la bague d'actionnement 60 atteignent l'extrémité des cames 44, opposée aux glissières rectilignes 43, le système chirurgical est dans la configuration de la figure 5. Dans cette configuration de la figure 5, les surfaces hélicoïdales 13 et 31 sont empêchées de se désengrener, en verrouillant ainsi la liaison hélicoïdale correspondante, et les faces d'appui 12 et 41 sont appuyées fermement l'une contre l'autre suivant la direction de l'axe X30. Ainsi, lorsque le mécanisme 50 est actionné suivant le second mouvement à la suite du premier mouvement, le mécanisme 50 verrouille la liaison hélicoïdale entre l'implant 10 et l'instrument 20, tout en pressant axialement les faces d'appui 12 et 41 l'une contre l'autre.

Tant que le système chirurgical 1 est dans la configuration de la figure 5, l'implant 10 est solidarisé fermement à l'instrument 20, en étant totalement immobilisé par rapport à ce dernier et en permettant la transmission de contraintes entre eux via l'interface formée par les faces d'appui 12 et 41. A titre d'exemple non limitatif, le chirurgien peut alors déplacer l'instrument 20 dans l'espace, de manière à implanter l'implant 10 dans le corps d'un patient, notamment en positionnant précisément l'implant 10 dans le corps du patient et, le cas échéant, en insérant en force ou en coinçant l'implant au niveau d'une structure anatomique du patient. A cet égard, dans l'exemple de réalisation envisagé aux figures, le support 40 comporte, dans sa région axialement opposée à la face d'appui 41, une face d'impaction 45 contre laquelle le chirurgien peut appliquer des efforts d'impaction aux fins de l'implantation de l'implant 10 dans le corps du patient.

Lorsque le chirurgien le souhaite, en particulier une fois que l'implant 10 est implanté dans le corps du patient, l'instrument 20 est déconnecté de l'implant 10, notamment sans modifier la position de ce dernier par rapport au corps du patient. Pour ce faire, le chirurgien actionne le mécanisme 50 de manière inverse à l'actionnement opéré pour connecter l'implant 10 et l'instrument 20 l'un à l'autre. Ainsi, le mécanisme 50 est d'abord actionné suivant un mouvement inverse au second mouvement précité, ce qui fait passer le système chirurgical de la configuration de la figure 5 à la configuration de la figure 4, puis le mécanisme 50 est actionné suivant un mouvement additionnel, inverse au premier mouvement précité, ce qui fait passer le système chirurgical 1 de la configuration de la figure 4 à la configuration de la figure 3. L'instrument 20 se retrouve ainsi dégagé de l'implant 10.

En tenant compte des explications données jusqu'ici, on comprend que le système chirurgical 1 est particulièrement facile à manipuler pour le chirurgien. De plus, lorsque le système chirurgical 1 est dans la configuration de la figure 5, la connexion entre l'implant 10 et l'instrument 20 est sécurisée. Par ailleurs, lorsque l'implant 10 et l'instrument 20 sont déconnectés, ils peuvent être facilement nettoyés et/ou décontaminés ; à cet effet, l'arbre 30, le support 40 et le mécanisme 50 peuvent être prévus démontables de manière simple.

Ces performances du système chirurgical 1 sont obtenues sans que ce dernier n'ait à recourir à aucune liaison filetée. Au contraire, la liaison entre l'implant 10 et l'instrument 20 est réalisée par la liaison hélicoïdale formée par les surfaces hélicoïdales 13 et 31, qui ne sont pas un filet et un taraudage d'une liaison filetée normalisée. Il en est de même pour la liaison hélicoïdale entre l'arbre 30 et le mécanisme 50, formée par les liaisons hélicoïdales 32 et 71. En particulier, suivant un dimensionnement préférentiel qui assure le fonctionnement du système chirurgical 1, tel que décrit plus haut, l'angle d'hélice de la liaison hélicoïdale entre l'implant 10 et l'instrument 20 et/ou de la liaison hélicoïdale entre l'arbre 30 et le mécanisme 50 est compris entre 6° et 45°, de préférence entre 10° et 40°, de préférence entre 15° et 45°. En pratique, l'angle d'hélice de la liaison hélicoïdale entre l'implant 10 et l'instrument 20 peut être identique ou différent de l'angle d'hélice de la liaison hélicoïdale entre l'arbre 30 et le mécanisme 50. Quand ces deux angles d'hélice sont différents, l'angle d'hélice de la liaison hélicoïdale entre l'implant 10 et l'instrument 20 est préférentiellement supérieur à l'angle d'hélice de la liaison hélicoïdale entre l'implant 10 et l'instrument 20. Dans tous les cas, la forme hélicoïdale non filetée des liaisons hélicoïdales précitées permet de réaliser par fabrication additive, typiquement par impression 3D, les surfaces hélicoïdales 13, 30, 31 et 71 et, plus généralement, une partie voire la totalité de l'implant 10 et/ou de l'instrument 20.

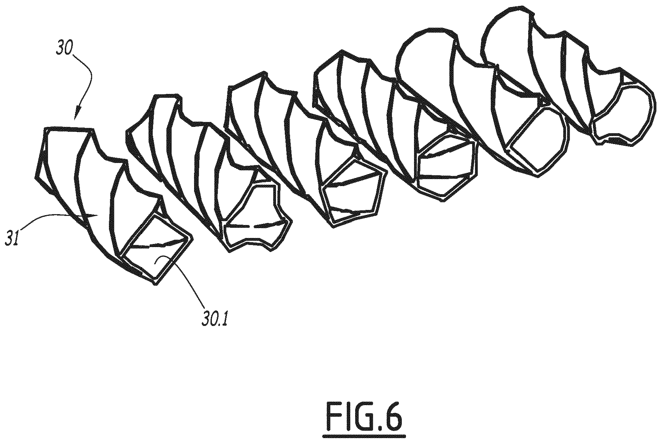

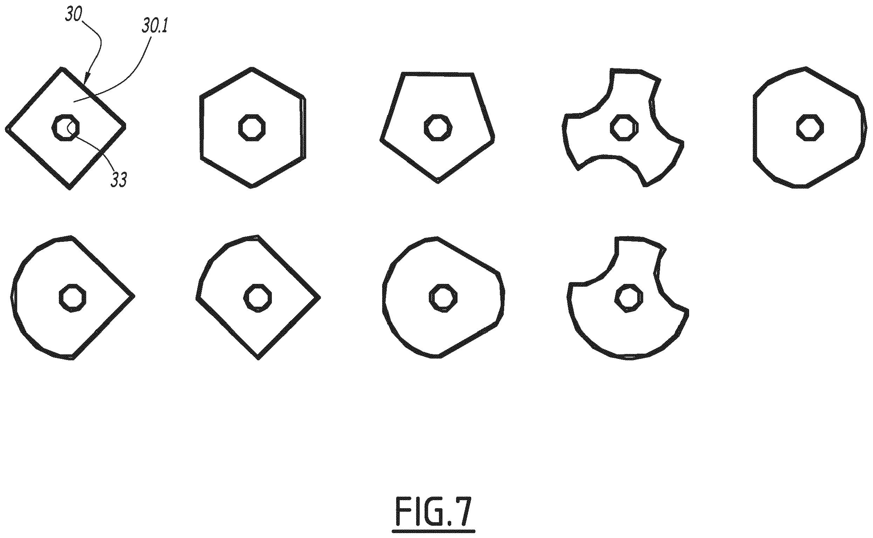

Dans le prolongement des considérations qui précèdent, les figures 6 et 7 illustrent la multitude de formes de réalisation que peut avoir la partie de l'arbre 30, délimitant la surface hélicoïdale 31, c'est-à-dire, ici, la partie longitudinale terminale 30.1 de cet arbre. Bien entendu, cette multitude de formes de réalisation se retrouve, par congruence, pour la partie de la cavité 14, délimitant la surface hélicoïdale 13. Ainsi, comme illustré par les figures 6 et 7, la section transversale de la partie de l'arbre 30, délimitant la surface hélicoïdale 31, présente avantageusement un contour non circulaire, comme montré sur les quatre premiers exemples de la figure 6, dans lesquels ce contour est respectivement carré, multilobé, pentagonal et hexagonal. Cette section transversale peut même présenter un contour asymétrique, comme illustré sur les deux derniers exemples de la figure 6 : cette asymétrie permet de détromper l'engagement de la surface hélicoïdale 31 avec la surface hélicoïdale 13. Par ailleurs, à titre d'aménagement optionnel illustré à la figure 7, l'arbre 30 peut être canulé, c'est-à-dire pourvu d'une canule 33 le traversant axialement de part en part.

Bien que non illustrées, des considérations similaires à celles développées dans le paragraphe précédent s'appliquent pour la partie de l'arbre 30, délimitant la surface hélicoïdale 32, et, par congruence, pour la partie du tiroir d'entraînement 70, délimitant la surface hélicoïdale 71.

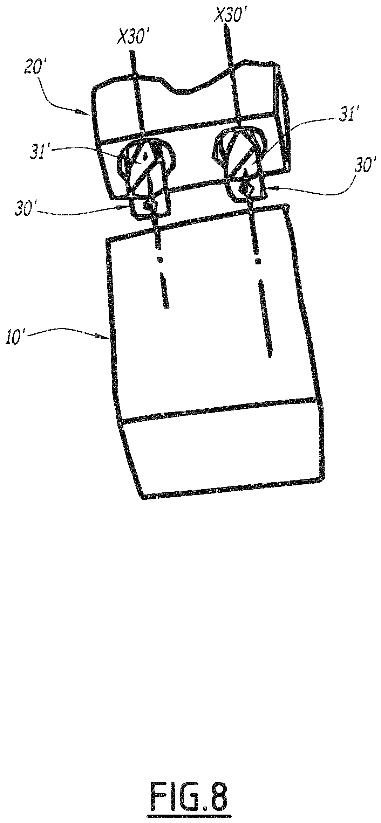

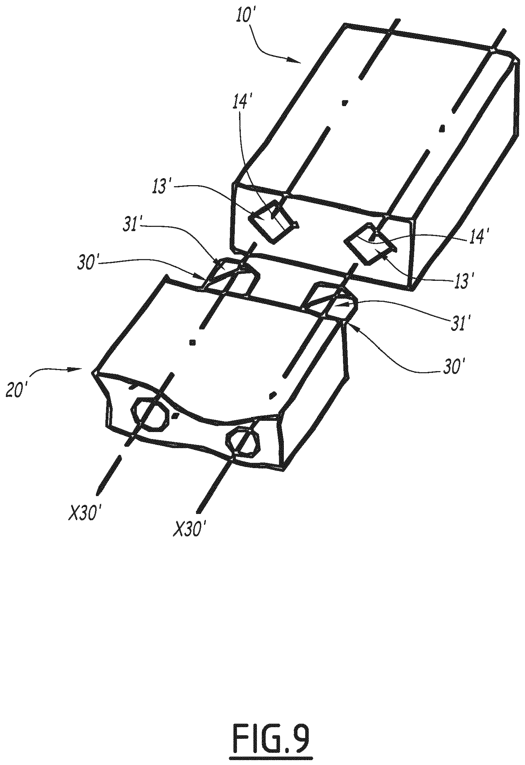

Sur les figures 8 et 9 est représenté un mode de réalisation alternatif au système chirurgical 1, qui est référencé 1'. Le système chirurgical 1' comprend un implant 10' et un instrument 20', qui sont fonctionnellement, voire structurellement similaires à l'implant 10 et à l'instrument 20, tout en se différentiant de ces derniers par la façon dont l'implant et l'instrument sont bloqués en rotation autour de l'axe X30 l'un par rapport à l'autre. Plus précisément, l'implant 10' et l'instrument 20' sont dépourvus de reliefs en creux et en saillie tels que les reliefs en creux 15 et en saillie 42 de l'implant 10 et de l'instrument 20. Dans le même temps, l'instrument 20' ne comporte pas qu'un seul arbre, tel que l'arbre 30, mais deux arbres 30', qui sont chacun similaires fonctionnellement, voire structurellement, à l'arbre 30 et dont les axes respectifs X30' sont parallèles l'un à l'autre. En particulier, chaque arbre 30' délimite une surface hélicoïdale 31' qui est fonctionnellement, voire structurellement similaire à la surface hélicoïdale 31 de l'arbre 30. De manière correspondante, l'implant 10' comporte deux cavités 14', qui sont chacune similaires fonctionnellement, voire structurellement, à la cavité 14 et dans chacune desquelles l'implant 10' délimite une surface hélicoïdale 13', qui est fonctionnellement, voire structurellement similaire à la surface hélicoïdale 13. Ainsi, les surfaces hélicoïdales 31' sont respectivement congruentes aux surfaces hélicoïdales 13 `et forment avec ces dernières deux liaisons hélicoïdales entre l'implant 10' et l'instrument 20', chacune de ces deux liaisons hélicoïdales étant fonctionnellement, voire structurellement similaires à la liaison hélicoïdale entre l'implant 10 et l'instrument 20. De par la présence de ces deux liaisons hélicoïdales, ces dernières bloquent en rotation autour de chacun des axes X30' l'implant 10' et l'instrument 20' l'un par rapport à l'autre lorsque les surfaces hélicoïdales 31' s'engrènent avec les surfaces hélicoïdales 13'.

En pratique, l'instrument 20' comporte un mécanisme, non-illustré sur les figures, qui est fonctionnellement similaire au mécanisme 50 de l'instrument 20, avec la spécificité avantageuse que ce mécanisme de l'instrument 20' est conçu pour agir en même temps et de la même manière sur les deux liaisons hélicoïdales entre l'implant 10' et l'instrument 20'.

Enfin, divers aménagements et variantes aux systèmes chirurgicaux 1 et 1' décrits jusqu'ici sont par ailleurs envisageables. A titre d'exemples :
- le rapport mâle/femelle entre l'implant 10 ou 10' et l'instrument 20 ou 20' peut être inversé ; il en est de même pour les reliefs en creux 15 et en saillie 42 ; et/ou
- d'autres types de fabrication que la fabrication additive peuvent être utilisés pour fabriquer tout ou partie de l'implant 10 ou 10' et/ou de l'instrument 20 ou 20' ; et/ou
- plutôt que le second mouvement précité combine des composantes rotative et translative, comme dans l'exemple décrit plus haut, ce second mouvement peut être prévu exclusivement rotatif, c'est-à-dire rotatif autour de l'axe X30, X30' sans être translatif suivant cet axe ; dans ce cas, le mécanisme 50 est adapté pour que son actionnement suivant ce second mouvement exclusivement rotatif verrouille la liaison hélicoïdale entre l'implant 10 et l'instrument 20, en empêchant le désengrènement des surfaces hélicoïdales 13 et 31, tout en faisant en sorte qu'à l'issue du second mouvement exclusivement rotatif, les faces d'appui 12 et 41 soient pressées axialement l'une contre l'autre, cette mise en appui des faces d'appui 12 et 41 étant soit atteinte à l'issue du premier mouvement et conservée lors du second mouvement, soit obtenue progressivement lors du second mouvement, dans les deux cas par le biais d'aménagements ad hoc du mécanisme ; et/ou
- plutôt que les deux éléments du système chirurgical 1 ou 1', connectables directement l'un à l'autre, soient un implant chirurgical et un instrument chirurgical, tels que l'implant 10 ou 10' et l'instrument 20 ou 20', ces deux éléments du système chirurgical peuvent être deux implants chirurgicaux qui sont par exemple à connecter directement l'un à l'autre au cours d'une intervention chirurgicale.

## Revendications

1. Système chirurgical (1 ; 1'), comprenant des premier (10 ; 10') et second (20 ; 20') éléments qui sont soit deux implants chirurgicaux, soit un implant chirurgical et un instrument chirurgical,
dans lequel le premier élément (10 ; 10') comporte :
- une première face d'appui (12), et
- une première surface hélicoïdale (13 ; 13') qui s'étend depuis la première face d'appui, et dans lequel le second élément (20 ; 20') comporte :
- une seconde face d'appui (41), qui est complémentaire de la première face d'appui (12),
- une deuxième surface hélicoïdale (31 ; 31'), qui s'étend depuis la seconde face d'appui et qui est congruente à la première surface hélicoïdale (13 ; 13') de manière que les première et deuxième surfaces hélicoïdales forment une première liaison hélicoïdale entre les premier et second éléments, centrée sur un axe (X30 ; X30'), et
- un mécanisme (50) pour commander la connexion réversible entre les premier et second éléments,
**caractérisé en ce que** le mécanisme (50) est adapté pour être actionné successivement suivant :
- un premier mouvement, qui est translatif suivant l'axe (X30 ; X30') sans être rotatif autour de l'axe et par lequel le mécanisme (50) fait s'engrener les première (13 ; 13') et deuxième (31 ; 31') surfaces hélicoïdales l'une avec l'autre jusqu'à juxtaposer axialement les première (12) et seconde (41) faces d'appui l'une contre l'autre, et
- un second mouvement, qui est rotatif autour de l'axe et par lequel le mécanisme verrouille la première liaison hélicoïdale alors que les première et second faces d'appui sont pressées axialement l'une contre l'autre.

2. Système chirurgical suivant la revendication 1, dans lequel la première liaison hélicoïdale entre les premier (10 ; 10') et second (20 ; 20') éléments présente un angle d'hélice compris entre 6° et 45°.

3. Système chirurgical suivant l'une des revendications 1 ou 2,
dans lequel la première surface hélicoïdale (13 ; 13') s'étend en creux dans la première face d'appui (12),
et dans lequel la deuxième surface hélicoïdale (31 ; 31') est agencée en saillie de la seconde face d'appui (41).

4. Système chirurgical suivant la revendication 3, dans lequel le second élément (20 ; 20') comporte :
- un arbre (30 ; 30'), qui est centré sur l'axe (X30 ; X30') et qui délimite extérieurement la deuxième surface hélicoïdale (31 ; 31'), et
- un support (40), qui délimite la seconde face d'appui (41), sur lequel l'arbre est monté à la fois de manière fixe suivant l'axe et de manière librement rotative autour de l'axe, et sur lequel le mécanisme (50) est monté mobile de façon à être actionnable suivant les premier et second mouvements et à entraîner l'arbre en rotation autour de l'axe par rapport au support lorsque le mécanisme est actionné en translation suivant l'axe.

5. Système chirurgical suivant la revendication 4,
dans lequel l'arbre (30) délimite extérieurement une troisième surface hélicoïdale (32), qui est distincte de la deuxième surface hélicoïdale (31),
et dans lequel le mécanisme (50) délimite une quatrième surface hélicoïdale (71) qui est congruente à la troisième surface hélicoïdale (32) de manière que les troisième et quatrième surfaces hélicoïdales forment une liaison hélicoïdale entre l'arbre (30) et le mécanisme.

6. Système chirurgical suivant l'une des revendications 4 ou 5,
dans lequel le mécanisme (50) comporte une bague d'actionnement (60), qui est guidée en déplacement par rapport au support (40) suivant les premier et second mouvements,
et dans lequel le mécanisme (50) comporte également un tiroir d'entraînement (70) qui est :
- lié en rotation autour de l'axe (X30) au support (40) tout en étant libre en translation suivant l'axe par rapport au support,
- lié en translation suivant l'axe à la bague d'actionnement (60) tout en étant libre en rotation autour de l'axe par rapport à la bague d'actionnement, et
- adapté pour entraîner l'arbre (30) en rotation autour de l'axe par rapport au support lorsque la bague d'actionnement est déplacée en translation suivant l'axe par rapport au support.

7. Système chirurgical suivant l'une quelconque des revendications 4 à 6, dans lequel l'arbre (30) présente, au moins dans sa partie longitudinale (30.1) délimitant la deuxième surface hélicoïdale (31), une section transversale dont le contour est non circulaire et, éventuellement, asymétrique.

8. Système chirurgical suivant l'une quelconque des revendications précédentes, dans lequel le second mouvement est, de manière combinée, rotatif autour de l'axe (X30 ; X30') et translatif suivant l'axe.

9. Système chirurgical suivant la revendication 8 prise en combinaison avec l'une quelconque des revendications 4 à 7, dans lequel le support (40) comporte au moins une came (44), qui s'enroule autour de l'axe (X30), de préférence avec un angle d'hélice supérieur à 84°, et qui guide en déplacement le mécanisme (50) suivant le second mouvement.

10. Système chirurgical suivant l'une quelconque des revendications précédentes, dans lequel les premier (10 ; 10') et second (20 ; 20') éléments coopèrent mécaniquement l'un avec l'autre de manière à être bloqués en rotation autour de l'axe (X30 ; X30') l'un par rapport à l'autre lorsque les première (13 ; 13') et deuxième (31 ; 31') surfaces hélicoïdales s'engrènent l'une avec l'autre.

11. Système chirurgical suivant la revendication 10, dans lequel l'un des premier (10) et second (20) éléments comporte au moins un relief en creux (15) tandis que l'autre des premier et second éléments comporte au moins un relief en saillie (42) qui coopère par complémentarité de formes avec le ou les reliefs en creux pour bloquer en rotation les premier et second éléments.

12. Système chirurgical suivant la revendication 10,
dans lequel les premier (10') et second (20') éléments forment une seconde liaison hélicoïdale entre eux, qui, conjointement à la première liaison hélicoïdale, bloque en rotation les premier et second éléments l'un par rapport à l'autre,
et dans lequel le mécanisme est conçu pour agir en même temps et de la même manière sur les première et seconde liaisons hélicoïdales entre les premier et second éléments.

## Patentansprüche

1. Chirurgisches System (1; 1'), umfassend ein erstes (10; 10') und ein zweites (20; 20') Element, die entweder zwei chirurgische Implantate oder ein chirurgisches Implantat und ein chirurgisches Instrument sind,
wobei das erste Element (10; 10') Folgendes umfasst:
- eine erste Anlagefläche (12) und
- eine erste schraubenförmige Oberfläche (13; 13'), die sich von der ersten Anlagefläche aus erstreckt, und wobei das zweite Element (20; 20') Folgendes umfasst:
- eine zweite Anlagefläche (41), die komplementär zu der ersten Anlagefläche (12) ist,
- eine zweite schraubenförmige Oberfläche (31; 31'), die sich von der zweiten Anlagefläche erstreckt und mit der ersten schraubenförmigen Oberfläche (13; 13') kongruent ist, sodass die erste und die zweite schraubenförmige Oberfläche eine erste schraubenförmige Verbindung zwischen dem ersten und dem zweiten Element bilden, die auf einer Achse (X30; X30') zentriert ist, und
- einen Mechanismus (50) zum Steuern der reversiblen Verbindung zwischen dem ersten und dem zweiten Element,
**dadurch gekennzeichnet, dass** der Mechanismus (50) angepasst ist, um nacheinander wie folgt betätigt zu werden:
- eine erste Bewegung, die translatorisch entlang der Achse (X30; X30') ist, ohne um die Achse drehbar zu sein, und durch die der Mechanismus (50) die erste (13; 13') und die zweite (31; 31') schraubenförmige Oberfläche ineinander greifen lässt, bis die erste (12) und die zweite (41) Anlagefläche axial nebeneinander sind, und
- eine zweite Bewegung, die um die Achse drehbar ist und durch die der Mechanismus die erste schraubenförmige Verbindung arretiert, während die erste und die zweite Anlagefläche axial gegeneinander gepresst werden.

2. Chirurgisches System nach Anspruch 1, wobei die erste schraubenförmige Verbindung zwischen dem ersten (10; 10') und dem zweiten (20; 20') Element einen Schraubenwinkel zwischen 6° und 45° aufweist.

3. Chirurgisches System nach einem der Ansprüche 1 oder 2,
wobei sich die erste schraubenförmige Oberfläche (13; 13') vertieft in die erste Anlagefläche (12) erstreckt,
und wobei die zweite schraubenförmige Oberfläche (31; 31') hervorstehend von der zweiten Anlagefläche (41) angeordnet ist.

4. Chirurgisches System nach Anspruch 3, wobei das zweite Element (20; 20') Folgendes umfasst:
- eine Welle (30; 30'), die auf der Achse (X30; X30') zentriert ist und die zweite schraubenförmige Oberfläche (31; 31') außen begrenzt, und
- einen Träger (40), der die zweite Anlagefläche (41) begrenzt, auf dem die Welle sowohl fest entlang der Achse als auch frei drehbar um die Achse montiert ist und auf dem der Mechanismus (50) beweglich montiert ist, um entlang der ersten und zweiten Bewegung betätigbar zu sein und die Welle um die Achse in Bezug auf den Träger zu drehen, wenn der Mechanismus entlang der Achse translatorisch betätigt wird.

5. Chirurgisches System nach Anspruch 4, wobei die Welle (30) außen eine dritte schraubenförmige Oberfläche (32) begrenzt, die von der zweiten schraubenförmigen Oberfläche (31) verschieden ist, und wobei der Mechanismus (50) eine vierte schraubenförmige Oberfläche (71) begrenzt, die mit der dritten schraubenförmigen Oberfläche (32) kongruent ist, sodass die dritte und die vierte schraubenförmige Oberfläche eine schraubenförmige Verbindung zwischen der Welle (30) und dem Mechanismus bilden.

6. Chirurgisches System nach einem der Ansprüche 4 oder 5, wobei der Mechanismus (50) einen Betätigungsring (60) umfasst, der in der ersten und der zweiten Bewegung in Bezug auf den Träger (40) beweglich geführt ist, und wobei der Mechanismus (50) auch einen Antriebsschieber (70) umfasst, der wie folgt ist:
- drehbar um die Achse (X30) verbunden mit dem Träger (40), während er entlang der Achse in Bezug auf den Träger frei translatorisch beweglich ist,
- translatorisch entlang der Achse verbunden mit dem Betätigungsring (60), während er in Bezug auf den Betätigungsring frei drehbar um die Achse ist, und
- angepasst, um die Welle (30) um die Achse in Bezug auf den Träger in Drehung zu versetzen, wenn der Betätigungsring entlang der Achse in Bezug auf den Träger translatorisch bewegt wird.

7. Chirurgisches System nach einem der Ansprüche 4 bis 6, wobei die Welle (30) zumindest in seinem Längsabschnitt (30.1), der die zweite schraubenförmige Oberfläche (31) begrenzt, einen Querschnitt aufweist, dessen Kontur nicht kreisförmig und möglicherweise asymmetrisch ist.

8. Chirurgisches System nach einem der vorherigen Ansprüche, wobei die zweite Bewegung auf kombinierte Weise um die Achse (X30; X30') drehbar und entlang der Achse translatorisch ist.

9. Chirurgisches System nach Anspruch 8 in Kombination mit einem der Ansprüche 4 bis 7, wobei der Träger (40) mindestens einen Nocken (44) umfasst, der die Achse (X30) vorzugsweise mit einem Schraubenwinkel von mehr als 84°, umläuft und den Mechanismus (50) in der zweiten Bewegung führt.

10. Chirurgisches System nach einem der vorherigen Ansprüche, wobei das erste (10; 10') und das zweite (20; 20') Element mechanisch miteinander zusammenwirken, um gegen eine Drehung um die Achse (X30; X30') in Bezug aufeinander arretiert zu werden, wenn die erste (13; 13') und die zweite (31; 31') schraubenförmige Oberfläche ineinander greifen.

11. Chirurgisches System nach Anspruch 10, wobei eines von dem ersten (10) und dem zweiten (20) Element mindestens ein vertieftes Relief (15) umfasst, während das andere von dem ersten und dem zweiten Element mindestens ein hervorstehendes Relief (42) umfasst, das formschlüssig mit dem oder den vertieften Reliefs zusammenwirkt, um das erste und das zweite Element gegen eine Drehung zu arretieren.

12. Chirurgisches System nach Anspruch 10, wobei das erste (10') und das zweite (20') Element eine zweite schraubenförmige Verbindung untereinander bilden, die zusammen mit der ersten schraubenförmigen Verbindung das erste und das zweite Element in Bezug aufeinander gegen eine Drehung arretiert,
und wobei der Mechanismus gestaltet ist, um gleichzeitig und auf die gleiche Weise auf die erste und die zweite schraubenförmige Verbindung zwischen dem ersten und dem zweiten Element zu wirken.

## Claims

1. A surgical system (1; 1'), comprising first (10; 10') and second (20; 20') elements which are either two surgical implants or a surgical implant and a surgical instrument,
wherein the first element (10; 10') includes:
- a first bearing face (12), and
- a first helical surface (13; 13') which extends from the first bearing face,
and wherein the second element (20; 20') includes:
- a second bearing face (41), which matches the first bearing face (12),
- a second helical surface (31; 31') which extends from the second bearing face and is congruent with the first helical surface (13; 13') so that the first and second helical surfaces form a first helical connection between the first and second elements, centered on an axis (X30; X30'), and
- a mechanism (50) for controlling the reversible connection between the first and second elements,
**characterized in that** the mechanism (50) is adapted to be actuated successively:
- along a first movement, which is a translation along the axis (X30; X30') without involving rotation about the axis and by which the mechanism (50) mates the first (13; 13') and second helical surfaces (31; 31') with each other until the first (12) and second bearing (41) faces are axially juxtaposed against each other, and
- along a second movement, which is a rotation about the axis and by which the mechanism locks the first helical connection while the first and second bearing faces are pressed axially against each other.

2. The surgical system of claim 1, wherein the first helical connection between the first (10; 10') and second (20; 20') elements has a helical angle comprised between 6° and 45°.

3. The surgical system according to one of claims 1 or 2, wherein the first helical surface (13; 13') extends recessed inside the first bearing face (12), and wherein the second helical surface (31; 31') is arranged protruding from the second bearing face (41).

4. The surgical system according to claim 3, wherein the second element (20; 20') includes:
- a shaft (30; 30') which is centered on the axis (X30; X30') and which externally delimits the second helical surface (31; 31'), and
- a support (40) which delimits the second bearing face (41), on which the shaft is mounted both in a fixed manner along the axis and so as to freely rotate about the axis, and on which the mechanism (50) is mounted movable so as to be apt to be actuated according to the first and second movements and to rotate the shaft about the axis with respect to the support when the mechanism is actuated in translation along the axis.

5. The surgical system according to claim 4, wherein the shaft (30) externally delimits a third helical surface (32), which is distinct from the second helical surface (31), and wherein the mechanism (50) delimits a fourth helical surface (71) which is congruent with the third helical surface (32) so that the third and fourth helical surfaces form a helical connection between the shaft (30) and the mechanism.

6. The surgical system according to one of claims 4 or 5, wherein the mechanism (50) includes an actuating ring (60), which is guided in motion with respect to the support (40), according to the first and second movements, and wherein the mechanism (50) further includes a drive sleeve (70) which is:
- linked in rotation to the support (40), about the axis (X30), while being free in translation along the axis with respect to the support,
- linked in translation to the actuating ring (60), along the axis, while being free to rotate about the axis with respect to the actuating ring, and
- suitable for rotating the shaft (30) about the axis with respect to the support when the actuating ring is moved in translation along the axis with respect to the support.

7. The surgical system according to any of claims 4 to 6, wherein the shaft (30) has, at least in the longitudinal part (30.1) thereof delimiting the second helical surface (31), a cross-section, the contour of which is non-circular and, potentially, asymmetrical.

8. The surgical system according to any of the preceding claims, wherein the second movement is, in a combined manner, a rotation about the axis (X30; X30') and a translation along the axis.

9. The surgical system according to claim 8 taken in combination with any of claims 4 to 7, wherein the support (40) includes at least one cam (44) which rolls around the axis (X30), preferentially with a helix angle greater than 84°, and which guides the mechanism (50) in motion following the second movement.

10. The surgical system according to any of the preceding claims, wherein the first (10; 10') and second (20; 20') elements mechanically cooperate with each other so as to be locked in rotation with respect to each other about the axis (X30; X30') when the first (13; 13') and second (31 ; 31') helical surfaces mate with each other.

11. The surgical system according to claim 10, wherein one of the first (10) and second (20) elements includes at least one recessed pattern (15) while the other of the first and second elements includes at least one protruding pattern (42) which cooperates by shape matching with the recessed pattern(s) so as to lock in rotation, the first and second elements.

12. The surgical system of claim 10, wherein the first (10') and second (20') elements form a second helical connection therebetween which jointly with the first helical connection, locks in rotation the first and second elements with respect to each other, and wherein the mechanism is suitable for acting simultaneously and in the same way on the first and second helical connections between the first and second elements.
